(19) Europäisches Patentamt
European Patent Office
Office européen des brevets

(11) **EP 2 811 292 A1**

(12) ## EUROPEAN PATENT APPLICATION
published in accordance with Art. 153(4) EPC

(43) Date of publication:
**10.12.2014 Bulletin 2014/50**

(21) Application number: **13742880.1**

(22) Date of filing: **29.01.2013**

(51) Int Cl.:
*G01N 27/447* (2006.01)    *C12M 1/00* (2006.01)
*C12Q 1/68* (2006.01)

(86) International application number:
**PCT/JP2013/051913**

(87) International publication number:
**WO 2013/115185 (08.08.2013 Gazette 2013/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.01.2012 JP 2012017325**

(71) Applicant: **Osaka University**
**Suita-shi, Osaka 565-0871 (JP)**

(72) Inventors:
• **KAWAI, Tomoji**
**Suita-shi,**
**Osaka 565-0871 (JP)**

• **TSUTSUI, Makusu**
**Suita-shi,**
**Osaka 565-0871 (JP)**
• **TANIGUCHI, Masateru**
**Suita-shi,**
**Osaka 565-0871 (JP)**

(74) Representative: **Jones, Helen M.M.**
**Gill Jennings & Every LLP**
**The Broadgate Tower**
**20 Primrose Street**
**London EC2A 2ES (GB)**

(54) ## CONTROL METHOD AND CONTROL DEVICE FOR MOVEMENT SPEED OF SUBSTANCE, AND USE THEREFOR

(57)    Provided are: a control method and control device for the movement speed of a substance which are capable of controlling the movement speed of the substance with good precision, and of raising the durability of the device; and a use therefor. An substance with charge is caused to move by a movement path formed by a first electrical field and a second electrical field that are formed in directions that intersect with each other.

FIG.1

**Description**

Technical Field

The present invention relates to a control method and a device to control movement speed of a substance and use thereof, and in particular relates to a control method and a device to control movement speed of a charged substance and use thereof.

Background Art

There is currently demand in various fields to develop technology capable of controlling the movement speed of a substance (for example a protein or nucleic acid) to a desired speed with good precision.

For example, in a sequencer for reading DNA base sequences, the DNA base sequences are read while the DNA is caused to move. When this is being performed, if the migration speed is too fast, then DNA base sequences cannot be determined with good precision. However, if the migration speed is too slow then an extremely long period of time is required to read the DNA base sequences. This means that there is also demand in the field of sequencing for technology capable of controlling the movement speed of a substance to a desired speed with good precision.

In particular, recently, there is rising interest in treatments tailor made to an individual's characteristics. In order to implement tailor made treatments, there is a need to read the base sequence of the genome of an individual person in a short period of time with good precision, and to accurately grasp the characteristic points of the base sequence of the genome of the individual. This may also be considered to be an urgent driver for development of technology capable of controlling the movement speed of a substance to a desired speed with good precision.

Due to the above circumstances, there is already technology employed to read nucleic acid base sequences by fixing a film of protein onto a lipid double layer and using electrophoresis to pass nucleic acids between a pair of electrodes (see, for example, Non-Patent Document 1). Specifically, in the technology employed in Non-Patent Document 1, the movement speed of nucleic acids is adjusted as electrophoresis is employed to pass nucleic acids through a pore formed by $\alpha$-heamolysin, and by measuring an ion current of this time, the base sequences of the nucleic acids are determined as they pass through the pore.

Related Art Publications

Non-Patent Documents

Non-Patent Document 1: Clarke, J., Wu, H.-C., Jayasinghe, L., Patel, A., Reid, S. and Bayley, H.; Continuous base identification for single-molecule nanopore DNA sequencing published in Nat. Nanotechnol. 4, 265-270 (2009).

SUMMARY OF INVENTION

Technical Problem

However, in the conventional technology described above, there is a problem in that it is difficult to control the movement speed of a single molecule with good precision. In particular, in conventional technology such as that described above, there is a problem in that it is difficult to slow the movement speed of a molecule.

Moreover, in the conventional technology as described above, there is a problem in that there is low device durability due to employing a protein as a material in the device.

In light of the above problems of conventional technology, an object of the present invention is to provide a control method and a device to control movement speed of a substance, so as to obtain good precision of control of movement speed of a substance and raised device durability, and usage thereof.

Solution to Problem

In order to solve the above problems, a control method of the present invention is a method of controlling movement speed of a substance, the method including: a movement process that moves a substance having a charge, along a first electrical field formed by a first electrode pair, wherein at least a portion of a movement path of the substance has a second electrical field formed by a second electrode pair in a direction intersecting with the first electrical field.

In order to solve the above problems, a control device of the present invention includes: a flow path provided between a first electrode pair; and a second electrode pair provided at at least a portion of the flow path, wherein a direction of a first electrical field formed by the first electrode pair and a direction of a second electrical field formed by the second electrode pair intersect with each other.

In order to solve the above problems, an apparatus to determine a nucleotide sequence of a polynucleotide of the present invention includes the control device of the present invention.

Advantageous Effects of Invention

The present invention exhibits the advantageous effect that the movement speed of a substance can be controlled with good precision. In particular, the present invention exhibits the advantageous effect that the movement speed of a substance can be decelerated.

Due to the present invention being able to control the movement speed of the substance with good precision, the advantageous effect that various measurements regarding the substance can be performed with good precision is exhibited. For example, taking the example of reading base sequences of DNA, since the present invention makes it possible to decelerate the movement speed of DNA, signals detected for each base configuring the DNA (for example a current signal or a fluorescent signal) can be prevented from overlapping with each other. As a result, the advantageous effect that DNA base sequences can be accurately determined is exhibited.

BRIEF DESCRIPTION OF DRAWINGS

Fig. 1 is diagram illustrating theory behind control in a control method for movement speed of the present invention.
Fig. 2 is a diagram illustrating Scanning Electron Microscope images of a control device of an Example of the present invention.
Fig. 3 is a diagram illustrating a state of a gap formed between positive and negative electrodes of a second electrode pair of an Example of the present invention.
Fig. 4 is a diagram illustrating a state of a voltage applied to a control device of an Example of the present invention and current flowing in the control device.
Fig. 5 is a graph illustrating a sealed state of a control device of an Example of the present invention.
Fig. 6(a) and (b) are graphs illustrating relationships, in a control device of an Example of the present invention, between ion current and time when $V_{long}$ = 0.5V is set as the voltage between Ag /AgCl electrodes and $V_{trans}$ = 0V is set as the voltage between Pt/ Au/ Pt/ $SiO_2$ nano-gap electrodes.
Fig. 7(a) and (b) are graphs illustrating relationships, in a control device of an Example of the present invention, between ion current and time when $V_{long}$ = 0.5V is set as the voltage between Ag /AgCl electrodes and $V_{trans}$ = 0.5V is set as the voltage between Pt/ Au/ Pt/ $SiO_2$ nano-gap electrodes.
Fig. 8 is a graph illustrating a distribution in substance movement times in a control device of an Example of the present invention.

DESCRIPTION OF EMBODIMENTS

Detailed explanation follows regarding exemplary embodiments of the present invention, however the present invention is not limited thereto. Note that references to "A to B" in the present specification mean "A to B inclusive".

1. Movement Speed Control Method

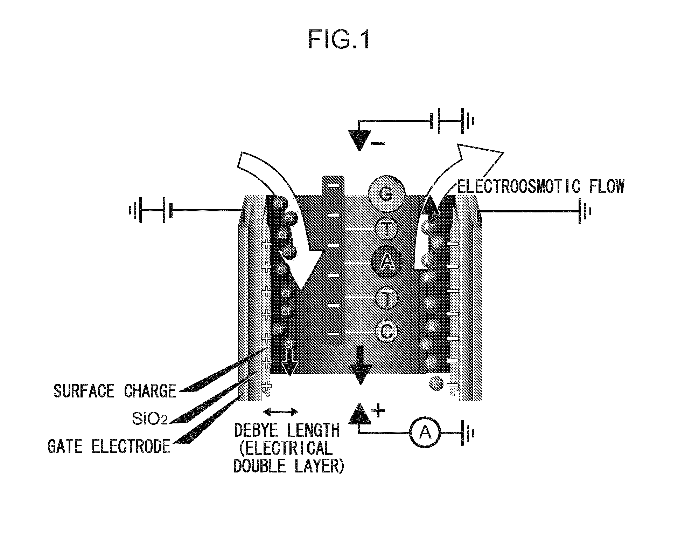

First, explanation follows regarding the theory behind control of the movement speed control method of the present invention, with reference to Fig. 1. Note that explanation follows regarding theory behind control with Fig. 1 as an example of a substance (specifically DNA) that has a negative charge, however it should be understood that, by reading the present specification, a person of skill in the art would also easily be able to employ the present invention to control movement speed of a substance with a positive charge.

As illustrated in Fig. 1, in the movement speed control method of the present invention, a charged substance is moved by a first electrode pair (an electrode pair provided at the top and bottom of the page in Fig. 1). Namely, the substance having a negative charge is moved from the negative electrode side of the first electrode pair (the top side of the page in Fig. 1) to the positive electrode side of the first electrode pair (the bottom side of the page in Fig. 1). This movement direction is the direction the substance is to be moved in, and the present invention controls the movement speed in this direction.

As illustrated in Fig. 1, a second electrode pair (an electrode pair provided at the left and right of the page in Fig. 1) is formed on the movement path of the substance separately to the first electrode pair.

Electrostatic interactions arise between the positive electrode of the second electrode pair and the negative charged substance. This enables the movement speed of the substance moving from the negative electrode side of the first electrode pair towards the positive electrode side of the first electrode pair to be decelerated using the electrostatic interactions.

When a substance having a positive charge is employed as the above substance, the substance moves from the positive electrode side of the first electrode pair (the bottom side of the page in Fig. 1) towards the negative electrode side of the first electrode pair (the top side of the page in Fig. 1).

In such cases, electrostatic interactions arise between the negative electrode of the second electrode pair and the positive charged substance. This enables the movement speed of the substance moving from the positive electrode side of the first electrode pair towards the negative electrode side of the first electrode pair to be decelerated using the electrostatic interactions.

When ions are present in the substance movement path, in addition to the deceleration effect of the electrostatic interactions described above, a further deceleration effect can be expected due to electroosmotic flow. Explanation follows regarding the deceleration effect due to electroosmotic flow.

As illustrated in Fig. 1, when there are anions (for example $Cl^-$) and cations (for example $K^+$) present in the substance movement path, electroosmotic flow due to the anions occurs at the surface of the positive electrode of the second electrode pair, and electroosmotic flow due to the cations occurs at the surface of the negative electrode of the second electrode pair. Note that the electroosmotic flow due to the anions is flow from the negative electrode side towards the positive electrode side of the first electrode pair, and the electroosmotic flow due to the cations is flow from the positive electrode side towards the negative electrode side of the first electrode pair.

When a negative charged substance is being moved, the electroosmotic flow occurring at the surface of the negative electrode of the second electrode pair is flow in the opposite direction to the substance movement direction. Consequently, separately to the electrostatic interaction described above, this enables the movement speed of the negative charged substance to be slowed by the electroosmotic flow.

However, when a positive charged substance is being moved, the electroosmotic flow occurring at the surface of the positive electrode of the second electrode pair is flow in the opposite direction to the substance movement direction. Consequently, this enables the movement speed of the positive charged substance to be slowed by the electroosmotic flow, separately to the electrostatic interaction described above.

Explanation follows regarding theory behind control of the movement speed control method of the above present invention, and explanation follows regarding exemplary embodiments of the movement speed control method based on the theory behind control.

The movement speed control method of the present exemplary embodiment includes a movement process that moves a charged substance along a first electrical field formed by the first electrode pair. Namely, in the movement speed control method of the present exemplary embodiment, a substance is move in a desired direction by electrostatic interaction between the first electrode pair and the charged substance, and a substance movement path is formed along a first electrical field formed by the first electrode pair (a field in the direction from the positive electrode side of the first electrode pair towards the negative electrode).

The substance may be any substance with charge, and there is no particular limitation to specific configurations thereof. For example, atoms, molecules, polymers, or composite bodies thereof may be employed. Making the above substance a substance that has a charge, not only enables the substance to be moved by the first electrode pair in the target direction, but it also enables the movement speed of the substance to be controlled by the second electrode pair.

The charged substance may have a negative charge, or may have a positive charge. The substance moves from the negative electrode side of the first electrode pair towards the positive electrode side when the charge possessed by the substance is a negative charge, and the substance moves from the positive electrode side of the first electrode pair towards the negative electrode side when the charge possessed by the substance is a positive charge.

When the above substance is a composite body of a substance A and a substance B, configuration may be made such that at least one of the substance A or the substance B has a charge. Obviously both the substance A and the substance B may be charged.

In cases in which both the substance A and the substance B are charged, the charge of the substance A and the charge of the substance B may both be positive charges or both negative charges, or one may be a positive charge and the other a negative charge. Configuration may be made such that the composite body has a charge when the composite body is viewed overall, even though the charges within the composite body partially cancel each other out. In other words, configuration may be made such that there is a certain degree of charge to enable movement of the composite body by the first electrode pair to obtained, and to enable control of the movement speed by the second electrode pair to be obtained.

When the above substance is a composite body of substance A and substance B, the substance A and the substance B may be joined together by sufficient force such that there is no separation during movement. For example, the substance A and the substance B may be joined towards through covalent bonds, ionic bonds, hydrogen bonds or hydrophobic bonds, or through plural bonds selected therefrom.

It is possible to employ as the substance A or the substance B, for example, an ionic surfactant (for example dodecyl-sulfate sodium salt), charged organic particles or charged inorganic particles. Since these substances are themselves charged, it is possible to impart charge to a composite body when forming the composite body with another substance. Namely, using these substances enables charged composite bodies to be formed easily.

The above ionic surfactant enables the desired substance to be readily contained within the self-forming micelle. This is because employing an ionic surfactant as one component to form a composite body enables a composite body with charge to be formed easily.

As is clear from the above explanation, the present invention enables movement speed to be controlled even for a substance that is not inherently charged, by forming a composite body with another substance that is charged.

Specific examples of the above charged substances are nucleic acids (DNA or RNA), amino acids, protein, pollen, virus, cells, organic particles or organic particles, however the present invention is not limited thereto.

There are no particular limitations to specific configurations of the above first electrode pair, and any suitable, known electrode pair may be employed therefor. Note that in the movement speed control method of the present exemplary embodiment, a movement path is formed for moving the substance between the negative electrode and the positive electrode of the first electrode pair.

There are no particular limitations to specific configurations of the above first electrode pair, and it is possible to employ any suitable, known electrodes therefor. For example, it is possible to employ Ag/ AgCl electrodes, however the present invention is not limited thereto.

There are no particular limitations to the separation between the positive electrode and the negative electrode of the first electrode pair, and any suitable setting may be employed. For example, suitable setting may be made in consideration of the charge of the substance, the voltage applied to the first electrode pair, and the length of the second electrode pair.

In the movement speed control method of the present exemplary embodiment, the second electrical field that intersects with the first electrical field is formed by the second electrode pair to at least a portion of the substance movement path. Namely, in the movement speed control method of the present exemplary embodiment, the first electrical field is formed by the first electrode pair from the positive electrode of the first electrode pair towards the negative electrode, and the second electrical field is formed by the second electrode pair from the positive electrode of the second electrode pair towards the negative electrode. The first electrical field and the second electrical field are also formed such that the first electrical field direction and the second electrical field direction intersect.

According to the above configuration, when the substance is moving due to the first electrical field formed by the first electrode pair, the substance passes through the second electrical field formed by the second electrode pair at least at a portion of the movement path. The movement speed of the substance is decelerated when moving through the second electrical field.

There are no particular limitations to the angle at which the first electrical field direction and the second electrical field direction intersect with each other, and any desired angle may be set. For example, it is possible to employ a configuration in which the first electrical field direction and the second electrical field direction are orthogonal to each other. Such a configuration enables an electrostatic interaction due to the second electrode pair to be caused to effectively act on the substance, and enables the substance to be efficiently decelerated. Moreover, such a configuration also enables an electroosmotic flow to be efficiently generated by the second electrode pair, and enables the substance to be efficiently decelerated by the electroosmotic flow.

A gap is formed between the positive electrode and the negative electrode of the second electrode pair, enabling part of the movement path to be formed by the gap. This enables the electrostatic interaction to be caused to act on the substance when the substance passes through the gap. There are no particular limitations to the size of such a gap formed in this manner, and setting may be made to any suitable, desired size.

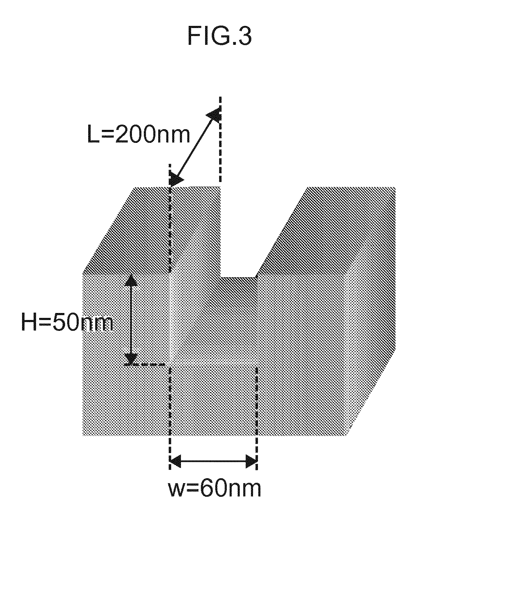

For example, Fig. 3 illustrates an example in which a gap is formed by facing negative and positive electrodes of the second electrode pair. In Fig. 3, the gap is formed as a cuboid body with width (W), height (H), and length (L). The width (W) corresponds to the distance between the negative and positive electrodes of the second electrode pair. The substance accordingly moves along the length (L). Namely, the substance moves from the back of the page of Fig. 3 towards the front of the page, or moves from the front of the page of Fig. 3 towards the back of the page. Note that although not illustrated in Fig. 3, the top side of the gap may be sealed off as required.

Each of the sizes of the width (W), the height (H) and the length (L) are not particularly limited, and may be set to a suitable desired size.

There are no particular limitations to the size of the width (W), and it may be appropriately set according to the type of substance, and whether a liquid or gel is disposed in the substance movement path. For example, setting may be made to 1nm to 1000nm, may be made to 1nm to 100nm, may be made to 1n to 60nm or may be made to 50nm to 60nm. Obviously the width (W) may be a size of 1nm or smaller, or may be a size of 1000nm or greater.

For example, since the molecular diameter of a nucleotide is known to a person of skill in the art, it is possible to set the size of the width (W) based on the molecular diameter. For example, since the molecular diameter of a nucleotide in a phosphate state is about 1nm, the width (W) may for example be set at 0.5nm to 2nm, at 1nm to 1.5 nm, or at 1nm to 1.2 nm.

The width (W) is preferably set narrow from the perspective of efficiently decelerating the substance.

Moreover, the size of the width (W) may be a size of magnitude such that electrical double layers that occur on the surfaces of the positive and negative electrodes of the second electrode pair (see Fig. 1) do not overlap with each other. Namely, if the thickness of the electrical double layers that occur at the surfaces of the positive and negative electrodes of the second electrode pair (the lengths in a direction connecting together the positive and negative electrodes of the second electrode pair) are designated "X" and "Y", and the width of the substance when moving between the positive and negative electrodes of the second electrode pair (the length in a direction connecting together the positive and negative electrodes of the second electrode pair) is designated "Z", then configuration may be made such that $W > X + Y + Z$.

Note that the thickness of the electrical double layer is known to depend on the concentration of ions forming the electrical double layer. It is accordingly possible to appropriately set "X" and "Y" according to the concentration of ions present in the movement path.

Moreover, in cases in which the shape of the above substance can be approximated to a straight chain, the length of the short direction of the straight chain can be made "Z". Obviously it is possible to make the length of the long direction of the straight chain "Z", or to make the average value of the length in the short direction and the length in the long direction of the straight chain "Z". It may be stated that, from the perspective of more certainly not overlapping the electrical double layer occurring at the surface of the positive and negative electrodes of the second electrode pair with the substance, it is preferably for the length of the long direction of the straight chain to be made "Z". Moreover, when the shape of the substance can be approximated to a sphere, the length of the diameter of the sphere may be made "Z". However, the above definitions of "Z" are merely examples thereof, and the present invention is not limited thereto.

As long as a configuration is adopted in which $W > X + Y + Z$ is satisfied, plural individuals of the substance may be moved, and the movement speed of these substances may be made to approximate more accurately to a normal distribution. Namely, according to such a configuration, the substance movement speed may be controlled with better precision.

The size of the height (H) is not particularly limited, and may be appropriately set according to the type of substance and whether a liquid or a gel is disposed in the movement path of the substance. For example, setting may be made to 1nm to 1000nm, may be made to 1nm to 100nm, may be made to 1n to 60nm or may be made to 50nm to 60nm. Obviously the height (H) may be a size of 1nm or smaller, or may be a size of 1000nm or greater.

It is possible to set the size of the height (H), similarly to the width (W) described above, at 0.5nm to 2nm, at 1nm to 1.5 nm, or at 1nm to 1.2 nm.

The size of the length (L) is not particularly limited, and may be appropriately set according to the type of substance and whether a liquid or a gel is disposed in the movement path of the substance. For example, setting may be made to 1nm to 1000nm, may be made to 100nm to 500nm, or may be made to 100n to 200nm. Obviously the length (L) may be a size of 1nm or smaller, or may be a size of 1000nm or greater.

It may be stated that the length (L) is preferably longer from the perspective of efficiently decelerating the substance speed.

There is no particular limitation to the voltage applied to the second electrode pair, and an appropriate desired voltage may be applied. For example, the voltage may be 0.10V to 1.00V, may be 0.25V to 0.75V, or may be 0.50V.

There is no particular limitation to specific configurations of the second electrode pair, and it is possible to employ any suitable known electrodes therefore. For example, it is possible to employ Pt/ Au/ Pt/ $SiO_2$ electrodes therefor, however the present invention is not limited thereto.

It is possible to dispose at least one of a liquid or gel in at least a portion of the movement path along which the substance moves. Obviously both a liquid and a gel may be disposed therein. Note that when at least one of a liquid or gel is disposed in the movement path, preferably placement is made within a gap formed by the positive and negative electrodes of the second electrode pair.

There is no particular limitation to such a liquid, and an example that may be given thereof is water. There is no particular limitation to such a gel, and examples that may be given thereof include a polyacrylamide gel, an agarose gel and the like.

It is preferable that at least ions with the opposite sign charge to the charge of the substance are contained in the liquid or gel. The above configuration enables current to flow between the first electrode pair and between the second electrode pair. This thereby enables changes in the current flowing between the first electrode pair to be detected with good sensitivity. Moreover, the above configuration enables electroosmotic flow to be generated at the surfaces of the positive and negative electrodes of the second electrode pair. This thereby enables the substance movement speed to be decelerated.

There is no particular limitation to specific configures of such ions. For example, KCl, NaCl or $CaCl_2$ may be dissolved in the liquid or gel disposed in the movement path. Preferably ions with a small valence are employed from out of the above from the perspective of generating a larger electroosmotic flow, specifically KCl and NaCl and the like.

There is no limitation to the concentration of ions dissolved in the liquid or gel, and it may for example be 0.01M to 1M, may be 0.1M to 0.5M, and may be 0.1M to 0.25M.

The movement speed control method of the present exemplary embodiment may include a detection process that separately detects plural normal distributions in substance movement speed when there are plural individuals of the substance moved. Note that there is no particular limitation to the number of substances moved, and any number may be employed therefor that enables statistical investigations to be made into the distribution of movement speeds.

As illustrated in Examples described later, movement speed of a substance can be decelerated by electrostatic interaction and electroosmotic flow, and when efficient deceleration is achieved by employing these means, the movement speeds of substance groups exhibit plural normal distributions. Namely, when using the above detection process plural normal distributions in the movement speed of substance groups are separately detected the this enables determination to be made that efficient deceleration of substance movement speed has been achieved.

In the above detection process, any process capable of separately detecting plural normal distributions in the movement speeds of substance groups may be employed, and there is no particular limitation to specific configurations thereof. For example, the above detection process may be a process that detects the moment when the value of current flowing between the first electrode pair drops, and measures the period of time over which the momentary drop occurs (the period of time during which the value of current drops), and statistically computes a distribution of movement speeds.

The above detection process may also include a selection process that selects a substance belonging to a desired normal distribution. The above selection process enables selection to be made of a substance group with a more appropriate movement speed. Note that there is no particular limitation to the normal distribution that the selection process selects, and it may be a normal distribution with a faster movement speed, or may be a normal distribution with a slower movement speed.

For example, consider a case in which DNA base sequences are read based on the movement speed control method of the present exemplary embodiment. When there are two types of movement speed present, then reading the base sequence of the DNA that moves with the slower movement speed enables the precision of DNA base sequence determination to be dramatically raised. However, reading the base sequence of the DNA that moves with the faster movement speed enables not only the precision of DNA base sequence determination to be raised in comparison to normal (for example in a configuration without the second electrode pair), but also enables the time required for reading to be shortened in comparison to cases in which the base sequence of the DNA that moves with the slower movement speed is read. Namely, providing a detection process and a selection process enables the base sequences of DNA to be read with the precision required and in the shortest possible time.

There is no particular limitation to configurations to implement the detection process and the selection process, and they may be implemented by employing a known current measurement device (such as for example an Axopatch 200B system manufactured by Molecular Devices LLC) and statistical processing software (such as for example Origin produced by Origin Lab Corporation).

2. Movement Speed Control Device

Explanation follows regarding a movement speed control device of the present exemplary embodiment. Note that in the following repetition of explanation common to the "1. Movement Speed Control Method" described above will be omitted.

A movement speed control device of the present exemplary embodiment includes a flow path provided between a first electrode pair, and a second electrode pair provided to at least a portion of the flow path. The direction of a first electrical field formed by the first electrode pair and the direction of a second electrical field formed by the second electrode pair intersect with each other. It may be stated that when this occurs, preferably the first electrical field direction and the second electrical field direction intersect with each other orthogonally.

There are no particular limitations to specific configurations of the above first electrode pair, and any suitable, known electrode pair may be employed therefor. For example, it is possible to employ Ag/ AgCl electrodes as the first electrode pair, however the present invention is not limited thereto.

There are no particular limitations to the separation between the positive electrode and the negative electrode side of the first electrode pair, and any suitable setting may be employed. For example, suitable setting may be made in consideration of the charge of the substance and of the voltage applied to the first electrode pair.

There are no particular limitations to the second electrode pair, and any suitable, known electrode pair may be employed therefor. For example, it is possible to employ Pt/ Au/ Pt/ SiO$_2$ electrodes as the second electrode pair, however the present invention is not limited thereto.

The above flow path may be broadly divided into a portion not sandwiched between the second electrode pair and a portion sandwiched between the second electrode pair. The portion sandwiched between the second electrode pair may be provided at any location on the flow path. For example, it may be provided at the head of the flow path, it may be provided partway along the flow path, or it may be provided towards the end of the flow path.

There is no particular limitation to the number of the second electrode pairs provided on the flow path, and one may be provided or plural may be provided. Providing plural of the second electrode pairs on a single flow path enables fine control of movement speed of the substance to be achieved. For example, when performing plural types of measurement related to the substance (for example a measurement A and a measurement B), it is possible to perform measurement A after adjusting the movement speed of the substance to an appropriate speed for measurement A using the second electrode pair disposed at the upstream side of the flow path, and then to perform measurement B after adjusting the movement speed of the substance to an appropriate movement speed for measurement B using a second electrode pair disposed at the downstream side of the flow path. The above configuration enables plural measurements to be performed under optimum conditions for each of the individual measurements whilst employing a single flow path. Note that there is no limitation particular limitation to the specific configurations of the above measurement A and measurement B, and examples that may be given thereof include measurement of ion current flow flowing between the second electrode pair, and measurement of fluorescent light emitted by the substance.

There are no particular limitations to the shape of the portion of the flow path not sandwiched between the second electrode pair, and any suitable desired shape may be employed. For example, a shape may be employed that enables the substance that has moved through the portion of the flow path not sandwiched between the second electrode pair to be guided into the portion sandwiched between the second electrode pair, and to enable the substance that has moved through the portion of the flow path sandwiched between the second electrode pair to be received.

For example, the cross-section of the portion of the flow path not sandwiched between the second electrode pair (a cross-section orthogonal to the substance movement direction) may be set with a width of 500nm to 1000nm and a height of 500nm to 1000nn, however there is no limitation thereto. The width may be set wider than the width of the gap between the positive and negative electrodes of the second electrode pair, and the height may be set higher than the height of the gap between the positive and negative electrodes of the second electrode pair.

There is no particular limitation to the shape of the portion of the flow path sandwiched between the second electrode pair, and it may be set appropriately. The shape of the portion of the flow path sandwiched between the second electrode pair corresponds to the "gap is formed by facing negative and positive electrodes of the second electrode pair" in the "1. Movement Speed Control Method", and details have already been explained with respect to Fig. 3. Further explanation thereof is accordingly omitted.

At least one of a liquid or gel containing at least ions with the opposite sign charge to the charge of the substance may be disposed in the flow path. The ions have already been explained with respect to the liquid or gel, and so explanation thereof is accordingly omitted.

The movement speed control device of the present exemplary embodiment may include a detection section (detection means) that separately detects plural normal distributions of movement speeds of the substances when there are plural individuals of the substance. Note that there is no particular limitation to the number of substances moved, and any number may be employed therefor that enables statistical investigations to be made into the distribution of movement speeds.

Separately detecting plural normal distributions in the movement speed of substance groups using the above detection section enables determination to be made as to whether or not efficient deceleration of the substance movement speed has been achieved.

The above detection section may employ any configuration capable of separately detecting plural normal distributions of the movement speeds of substance groups, and there is no particular limitation to specific configurations thereof. For example, the above detection section may be configured to detect moments when the value of current flowing between the first electrode pair drops, and to measure the period of time of the momentary drop (the period of time during which the value of current drops), and to statistically compute a distribution of movement speeds of the substances from the measured periods of time.

The above detection section may also include a selection section (selection means) that selects a substance belonging to a desired normal distribution. The above selection section enables selection to be made of a substance group with a more appropriate movement speed. Note that there is no particular limitation to the normal distribution that the selection section selects, and it may be a normal distribution with a faster movement speed, or may be a normal distribution with a slower movement speed.

There is no particular limitation to specific configurations to implement the detection section and the selection section, and they may be implemented by employing a known current measurement device (such as for example an Axopatch 200B system manufactured by Molecular Devices LLC) and statistical processing software (such as for example Origin produced by Origin Lab Corporation).

3. Polynucleotide Nucleotide Sequence Determination Apparatus

A polynucleotide nucleotide sequence determination apparatus of the present exemplary embodiment includes the control device of the present invention. Since explanation has already been given of the control device of the present invention, explanation follows regarding other parts of the configuration.

The polynucleotide nucleotide sequence determination apparatus of the present exemplary embodiment may be configured by combining a known sequencer with the control device of the present invention. Note that configuration components of the polynucleotide may be DNA or may be RNA.

For example, in one type of known sequencer, whilst groups of DNA fragments stained with a fluorescent dye are being separated by a gel (for example by a plate shaped gel or a capillary shaped gel), the base sequence thereof is read by detecting the type of fluorescent dye attached to each of the DNA fragments.

In such cases, configuration may be made by disposing the control device of the present invention in at least one portion of a movement path of DNA fragments formed by a gel, and disposing a configuration to detect fluorescence at the downstream side of the control device. According to such a configuration, the movement speed of the DNA fragments is decelerated when the fluorescent dye stained DNA fragments pass through between the second electrode pair, and then the fluorescence is detected afterwards. As a result, the base sequence may be determined with good precision.

Moreover, it is possible to configure the polynucleotide nucleotide sequence determination apparatus of the present invention by combining the "polynucleotide nucleotide sequence determination apparatus" described in PCT/JP2011/054631 with the control device of the present invention.

In such cases, similar to in the case described above, a configuration to read base sequences may be disposed on the movement path of the DNA fragment at the downstream side of the second electrode pair.

Note that PCT/JP2011/054631 is incorporated by reference in the present specification.

Examples

1. Control Device Fabrication

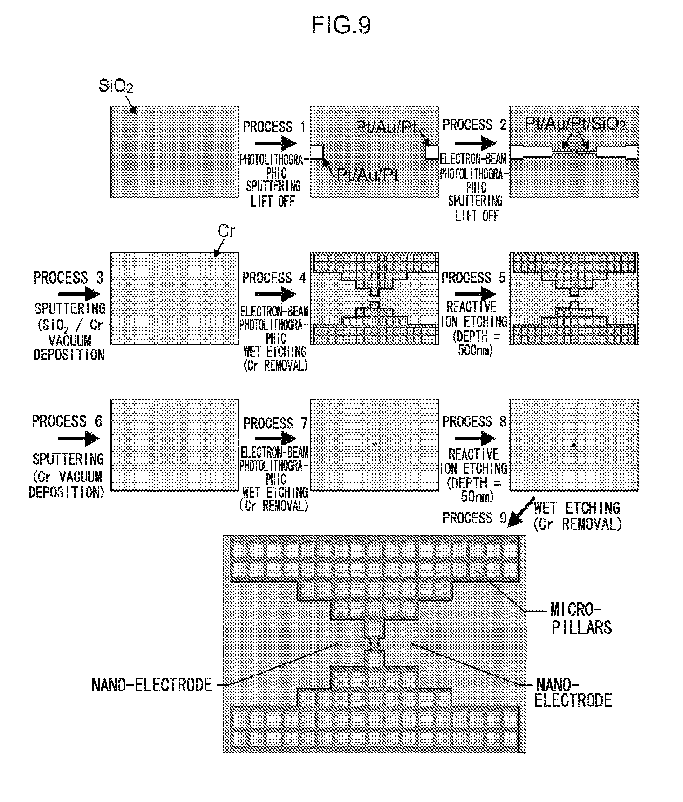

A control device of the present Example is fabricated by the following processes 1 to 9. Explanation follows regarding a fabrication method of the control device of the present exemplary embodiment, with reference to Fig. 9. Note that Fig. 9 is a plan view of a control device in each of the processes. The following processes 1 and 2 are processes for fabricating Pt/ Au/ Pt/ $SiO_2$ nano-gap electrodes on a substrate, and process 3 to process 9 are processes for fabricating a flow path on a substrate.

Process 1

An extraction electrode is patterned using photolithography on a doped Si wafer covered with a $SiO_2$ thermal oxidation coating film of 300nm thickness (resist: AZ5206E).

Then, a Pt(2nm)/ Au(20nm)/ Pt (2nm) film is stacked thereon by metal vapor deposition using a high frequency magnetron sputtering method.

After immersing the above substrate in N, N- dimethylformamide for 8 hours, the Pt/Au/Pt extraction electrode is produced by performing lift-off of resist on the substrate using ultrasonic cleaning.

Process 2

The nano-gap electrodes are plotted by an electron-beam lithography method using external marks made in the vicinity of the Pt/ Au/ Pt extraction electrode as an index (resist: ZEP520A-7; inter electrode distance 50nm).

Then Pt(2 nm)/ Au(30nm)/ Pt(2 nm)/ $SiO_2$ (50nm) stacked layers are vapor-deposited thereon using a high frequency magnetron sputtering method.

After immersing the above substrate in N, N- dimethylformamide for 8 hours, the Pt/Au/ Pt/ $SiO_2$ nano-gap electrode is produced by performing a liftoff process using ultrasonic cleaning.

Process 3

An $SiO_2$ (15 nm)/ Cr (100nm) film is vapor-deposited over the entire substrate using a high frequency magnetron sputtering method.

Process 4

A square pattern layout is plotted in the vicinity of the Pt/ Au/ Pt/ $SiO_2$ nano-gap electrodes using an electron-beam lithography method with the external marks used in process 2 as an index (resist: ZEP520A-7).

Then resist is removed and a Cr layer exposed thereby is removed by immersing the substrate in Cr etching solution (room temperature for 60 seconds).

Process 5

The remaining Cr layer is used as a mask and the $SiO_2$ layer exposed by a reactive ion etching method is cut-down in the depth direction to 500nm. Micro flow paths are thereby fabricated of 500nm high pillars arranged in a row.
After performing ion etching, a Cr etching solution is used to remove the remaining Cr layer.

Process 6

A 25nm thick Cr film is vapor-deposited on the substrate using a high frequency magnetron sputtering method.

Process 7

A flow path of width 500nm is plotted on the electrodes using superimposed plotting using an electron-beam lithography method (resist: ZEP520A-7).
Cr of the portion to be the flow path is removed by immersing the above substrate sample in a Cr etching solution.

Process 8

A flow path in which Pt/ Au/ Pt/ $SiO_2$ nano-gap electrodes are buried is produced by cutting the exposed $SiO_2$ down to a depth of 50nm using a reactive-ion etching method ($CF_4$, 4.2 Pa, 100W).

Process 9

Finally, the remaining Cr layer is removed using Cr etching solution. When this is performed it is designed to leave a 15nm thick $SiO_2$ layer remaining at an upper portion of the Pt/ Au/ Pt/ $SiO_2$ nano-gap electrodes. Thereby an upper portion of the Pt/ Au/ Pt/ $SiO_2$ nano-gap electrode can be sealed over by a PDMS (Polydimethylsiloxane) block used to seal the flow path at a later stage (PDMS does not readily adhere to metal).

Pretreatment

In the control device fabricated by the processes 1 to 9, pretreatment is performed before actual use. Explanation follows regarding specific contents of such pretreatment.
When actually using the control device, the upper portion of the fabricated control device may be sealed by a PDMS block. The above configuration enables liquid to be prevented from leaking from the flow path.
A micro flow paths are made in the PDMS block on the surface at the side mounted to the substrate. The production method is as follows.
First, a mold is manufactured for producing the micro flow path of the PDMS block. Specifically, photoresist SU-8-3050 (layer thickness $200\mu$m) is coated on a Si wafer, and then heated for 45 minutes at 90°C. After the solvent in the SU-8-3050 has evaporated, the substrate is gradually cooled to room temperature over a period of 1 hour.
A flow path pattern of 0.4mm width is plotted on the above substrate using photolithography. Then, the light-exposed portion of the SU-83050 is removed by immersing in a developing liquid for SU-8-3050, and a mold is produced.
Then, PDMS (Sylgard (registered trademark) 184) is flowed onto the mold placed in a petri dish, and the PDMS is cured by heating for 1 hour at 70°C.
The cured PDMS is cut up into 20mm size squares, thereby obtaining PDMS blocks with flow paths.
In order to seal the flow paths in the PDMS blocks, oxygen plasma treatment is performed to the surface of the PDMS on the side to be adhered, and to the surface of the flow path device (50W for 45 seconds).
Then, the PDMS block and the $SiO_2$ are adhered together by promptly placing the above surfaces together, sealing the flow path. At this stage there are already six through holes that have been formed in advance in the PDMS block. Two of these through holes are used to insert the Ag/ AgCl electrodes for measuring the ion current (of the $I_{ion}$ described later) flowing through the flow path, and other four through holes are used as inlet ports for introducing liquid containing molecules.

2. Confirmation of Shape and Size of Gap Between Second Electrode Pair

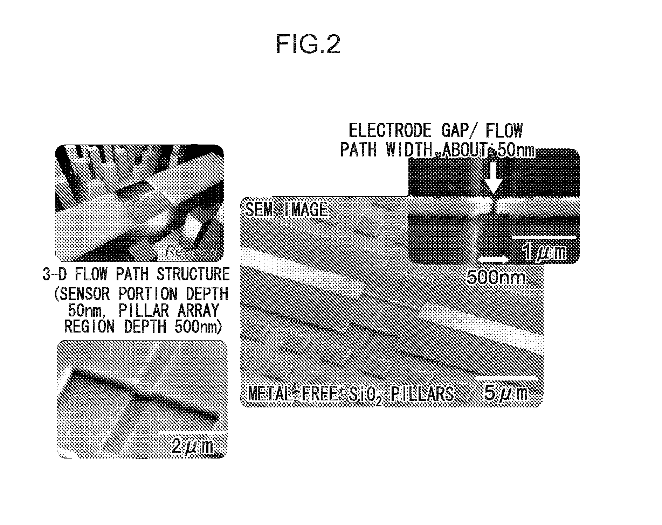

In order to confirm the shape of the control device produced by "1. Control Device Fabrication", the fabricated control device is observed with a scanning electron microscope. Fig. 2 illustrates schematic images of the fabricated control device as observed with a scanning electron microscope, and Fig. 3 schematically illustrates a structure of a gap formed by facing Pt/ Au/ Pt/ SiO$_2$ nano-gap electrodes (corresponding to the second electrode pair).

As shown in Fig. 3, the gap formed by the facing Pt/ Au/ Pt/ SiO$_2$ nano-gap electrodes is about 200nm in length (L), about 50nm in height (H), and about 60nm in width (W).

3. Confirmation of Sealed State

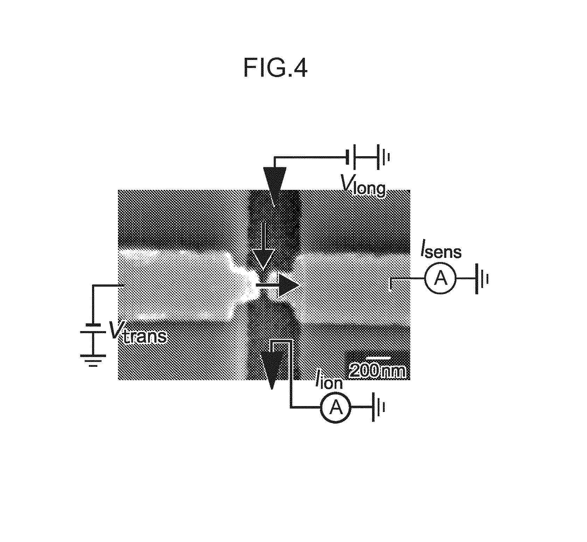

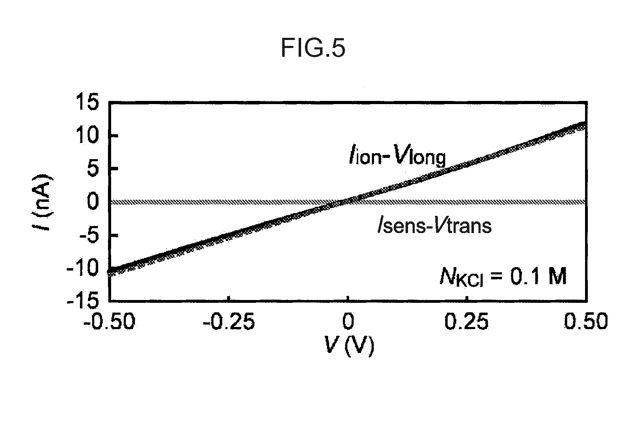

The control device fabricated by "1. Control Device Fabrication" is then checked to see whether it is sealed by the PDMS block. The checking method and results are explained below, with reference to Fig. 4 and Fig. 5.

ATE buffer (KCl: 0.1M, tris-HCl:10mM, EDTA:1mM) solution is introduced into the flow paths on both sides of the control device fabricated by "1. Control Device Fabrication". After filling with TE buffer solution, the Ag/AgCl electrodes for measuring the ion current (I$_{ion}$ of Fig. 4) flowing through the flow paths are inserted to both sides of the flow path.

The Ag/AgCl electrodes are fabricated by coating Pt wire of 0.1 mm diameter with an Ag/AgCl paste (BAS) and then heating for 10 minutes or longer at 100°C.

To measure ion current, a direct current voltage of 0.5V (V$_{long}$ of Fig. 4) is applied to one of the Ag/AgCl electrodes inserted into the flow path, and the electric current (I$_{ion}$ of Fig. 4) which flows to the Ag/AgCl electrode on the opposite side of the electrode pair is recorded at a sampling rate of 1 MHz using a high-speed digitizer (NI-PXI-5922) through a high-speed current amplifier with 10$^8$ A/V gain. Note that in this test a direct current voltage is not applied to the Pt/ Au/ Pt/ SiO$_2$ nano-gap electrodes (V$_{trans}$ = 0 in Fig. 4).

The solid line in Fig. 5 shows the value of I$_{ion}$ (see I$_{ion}$ -V$_{long}$ of Fig. 5) and the value of I$_{sens}$ (refer to I$_{sens}$ -V$_{trans}$ of Fig. 5) detected in the above test.

However, the value of I$_{ion}$ can be derived theoretically using the following Equation (1). Namely,

$$I_{ion} = N_{KCl} \times \mu \times V_{long} \times A/L \tag{1}$$

In the above Equation (1), N$_{KCl}$ represents the ion concentration, $\mu$ represents the ion mobility, A represents the channel cross-sectional area (specifically corresponding to H $\times$ W in Fig. 3), L represents the channel length (specifically corresponding to L in Fig. 3). Values of I$_{ion}$ theoretical derived according to Equation (1) are illustrated by the broken line on Fig. 5.

It is clear from Fig. 5 that the values of the I$_{ion}$ detected in the actual test and the theoretically derived values of the I$_{ion}$ substantially match each other. This accordingly indicates that the flow path of the control device fabricated by "1. Control Device Fabrication" is sealed by the PDMS block.

4. Movement Speed Measurement

4-1. Measurement Method

Investigation is performed into whether or not there is a change in the movement speed of a substance that moves in the flow path when a direct current voltage is not applied to the Pt/ Au/ Pt/ SiO$_2$ nano-gap electrodes, and when a direct current voltage is applied to the Pt/ Au/ Pt/ SiO$_2$ nano-gap electrodes.

For the flow path of the control device fabricated by "1. Control Device Fabrication", a solution containing biomolecules is flowed into the flow path through holes open in the PDMS block.

Specifically, a TE buffer (KCl: 0.1M, tris-HCl: 10mM, EDTA: 1mM) solution containing λ-DNA (Takara Bio) at a concentration of 10nM is introduced into the one side of the flow path, and a TE buffer (KCl: 0.1M, tris-HCl: 10mM, EDTA: 1mM) solution not containing λ-DNA (Takara Bio) is introduced into the flow path on the other side.

After filling the TE buffer solution, Ag/AgCl electrodes are inserted into both sides of the flow path. The Ag/AgCl electrodes are fabricated by coating Pt wire of 0.1 mm diameter with an Ag/AgCl paste (BAS) and then heating for 10 minutes or longer at 100°C.

To measure the ion current, a direct current voltage of 0.5V is applied to one of the Ag/AgCl electrodes inserted into the flow path, and the electric current which flows to the Ag/AgCl electrode on the opposite side of the electrode pair is recorded at a sampling rate of 1 MHz using a high-speed digitizer (NI-PXI-5922) through a high-speed current amplifier with 10$^8$ A/V gain.

4-2. Test Result

A. Result 1

The voltage between the Ag/AgCl electrodes is set to $V_{long}$ = 0.5V, the voltage between the Pt/ Au/ Pt/ SiO$_2$ nano-gap electrodes is set to $V_{trans}$ = 0V, and changes with time are measured in the ion current ($I_{ion}$) which flows through the flow path under the condition that λ-DNA is present. Not that, as a comparision, changes with time are also measured in the ion current ($I_{ion}$) which flows through the flow path under the condition that λ-DNA is not present.

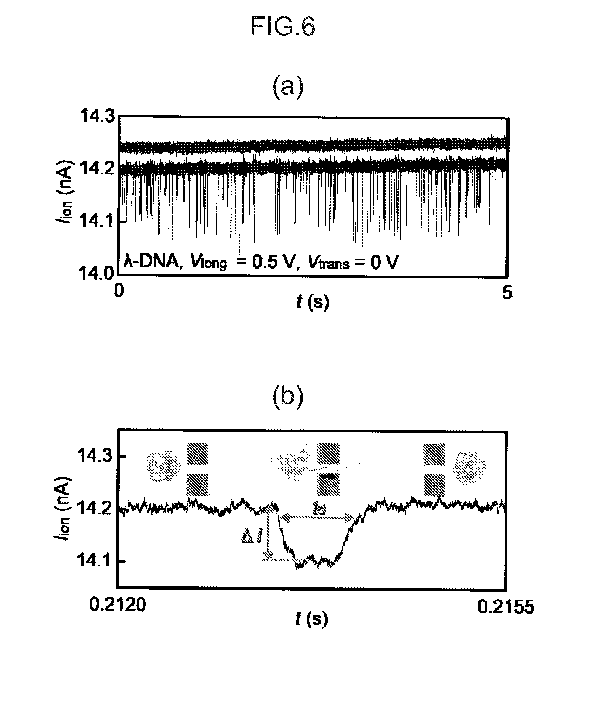

Results from the above tests are illustrated in Fig. 6(a). The data at the top of Fig. 6(a) illustrates values of the ion current ($I_{ion}$) measured under the condition that λ-DNA is not present, and the data at the bottom of Fig. 6(a) illustrates values of the ion current ($I_{ion}$) measured under the condition that λ-DNA is present.

As shown in the data at the top of Fig. 6(a), the values of the ion current ($I_{ion}$) measured under the condition that λ-DNA is not present shows no change. As shown in the data at the bottom of Fig. 6(a), the values of the ion current ($I_{ion}$) measured under the condition that λ-DNA is present shows a tendency to decreases in a spike shape.

The above spike shaped changes in the current are changes that arise when one molecule of λ-DNA passes through the portion where a gap is formed by the facing Pt/ Au/ Pt/ SiO$_2$ nano-gap electrodes, and impedes the flow of ions along the gap. Fig 6(b) illustrates an enlargement of a spike shaped change in current.

As illustrated in Fig. 6(b), the $I_{ion}$ values drop by ΔI (nA) while the λ-DNA is passing between the gap formed by the facing Pt/ Au/ Pt/ SiO$_2$ nano-gap electrodes (during the period of time td(s). This illustrates that the magnitude of the speed of the λ-DNA can be determined by measuring td(s). Namely, when comparing td(s) for the same substance against each other, larger values of td(s) indicate that the movement speed of the substance is slower.

Note that from Fig. 6(b) the average td(s) value is 0.0005 seconds when the voltage between the Ag/AgCl electrodes is $V_{long}$ = 0.5V and the voltage between the Pt /Au/ Pt/ SiO$_2$ nano-gap electrodes is $V_{trans}$ = 0V.

B. Result 2

The voltage between the Ag/AgCl electrodes is set to $V_{long}$ = 0.5V, the voltage between the Pt/ Au/ Pt/ SiO$_2$ nano-gap electrodes is set to $V_{trans}$ = 0.5V, and changes with time are measured in the ion current ($I_{ion}$) which flows through the flow path under the condition that λ-DNA is present.

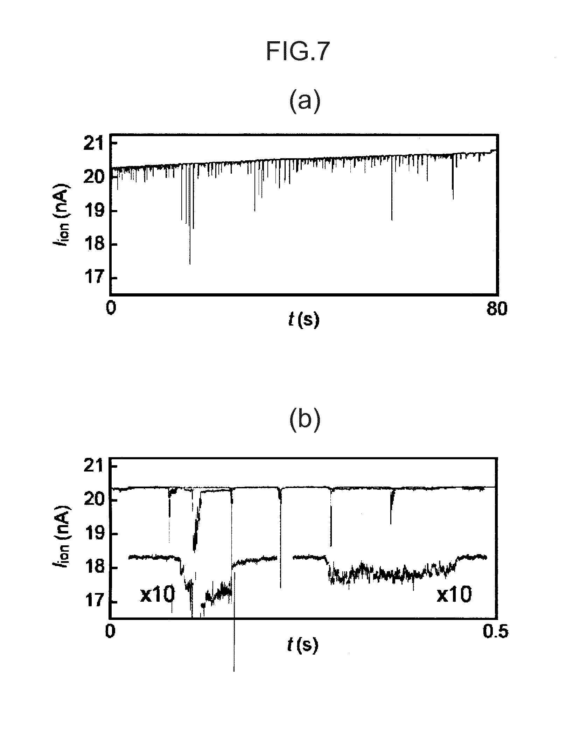

Results from the above tests are illustrated in Fig. 7(a) and (b). Note that data at the bottom of Fig. 7(b) is an enlargement of part of the data of Fig. 6(a).

As shown in the data of Fig. 7(a) and (b), there are two types of spike shaped current changes observed when a voltage is applied between the Pt/ Au/ Pt/ SiO$_2$ nano-gap electrodes. Respective values of 0.006 seconds and 0.2 seconds are computed as average td(s) values for these spike shaped current changes. The data illustrates that the substance movement speed shows a deceleration to about 1/10 to about 1/400 of the movement speed when no voltage is applied between the Pt/ Au/ Pt/ SiO$_2$ nano-gap electrodes.

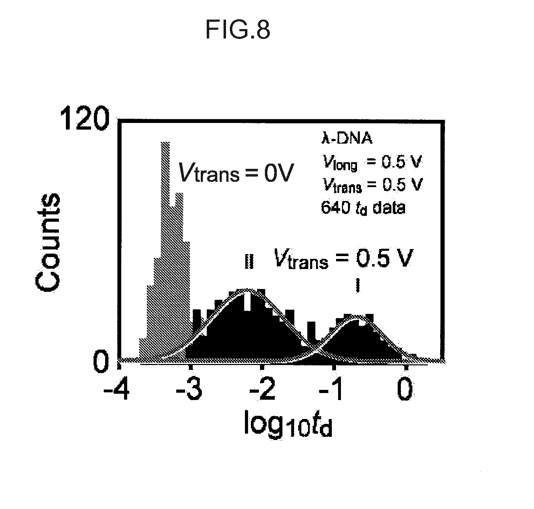

The distribution of the td(s) values when a voltage is not applied between the Pt/ Au/ Pt/ SiO$_2$ nano-gap electrodes, and the distribution of the td(s) values when a voltage is applied between the Pt/ Au/ Pt/ SiO$_2$ nano-gap electrodes, are illustrated in Fig. 8. Note that the distributions I and II illustrated at in Fig. 8 of the td(s) values are distributions of td(s) values when a voltage is applied between the Pt/ Au/ Pt/ SiO$_2$ nano-gap electrodes.

It is clear from Fig. 7 and Fig. 8 that the substance movement speed can be slowed both when a voltage is applied between the Pt/ Au/ Pt/ SiO$_2$ nano-gap electrodes, and when a voltage is not applied between the Pt/ Au/ Pt/ SiO$_2$ nano-gap electrodes. Moreover, it is clear that the substance movement speed is adjustable obtain two types of movement speed when the voltage is applied between the Pt/ Au/ Pt/ SiO$_2$ nano-gap electrodes.

Note that it is thought that two types of speed described above are caused by the electrostatic states that arise on the wall faces of the flow path due to voltage being applied between the Pt/ Au/ Pt/ SiO$_2$ nano-gap electrodes.

As illustrated in Fig. 1, the negative charged DNA is electrostatically pulled to the wall face of the flow path on the positive electrode side, slowing the electrophoretic speed of the DNA due to electrostatic force between the DNA and the electrodes. However, due to an electrical double layer of positive ions (K$^+$ ions) being formed in the solution in the vicinity of the wall face of the flow path on the negative electrode side, electroosmotic flow is induced in the opposite direction to the DNA migration speed. The migration speed of the DNA is accordingly slowed due to the influence of the electroosmotic flow. It is accordingly thought that the two types of speed distribution can be observed according to which side the DNA flows on out of the positive electrode or the negative electrode sides.

Since a strong electrostatic interaction arising between the electrode and the molecules on the positive electrode side, and a large accompanying effect due to intermolecular forces between the gold and the DNA, would be expected, the large deceleration to about 1/400 is thought to be a phenomenon that arises when the DNA flows on the positive electrode side of the Pt/ Au/ Pt/ SiO$_2$ nano-gap electrodes. The deceleration to about 1/10 is thought to be a phenomenon that arises when the DNA flows on the negative electrode side of the Pt/ Au/ Pt/ SiO$_2$ nano-gap electrodes

The present invention is not limited by each of the configurations explained above, and various modifications are possible within a range defined by the scope of the patent claims, and exemplary embodiments obtained by appropriate combination of the technical means described herein in each of the different respective exemplary embodiments and examples are included in the technical scope of the present invention.

In order to address the above issues, a control method of the present invention includes a movement process that moves a substance with a charge along a first electrical field formed by a first electrode pair, wherein in the substance movement speed control method, at least a portion of the substance movement path has a second electrical field formed by a second electrode pair in a direction intersecting with the first electrical field.

In the control method of the present invention, preferably the first electrical field direction and the second electrical field direction intersect with each other orthogonally.

In the control method of the present invention, preferably the substance is moved through at least a liquid or a gel containing at least an ion with an opposite charge to the charge of the substance.

The control method of the present invention preferably further includes a detection process that separately detects plural normal distributions of movement speed of the substance when plural individuals of the substance are moved.

In the control method of the present invention, preferably the substance is a nucleic acid, a protein, a pollen, a virus, a cell, an organic particle or an inorganic particle.

In order to address the above issues, a control device of the present invention includes: a flow path provided between a first electrode pair; and a second electrode pair provided to at least a portion of the flow path, wherein a direction of a first electrical field formed by the first electrode pair and a direction of a second electrical field formed by a second electrode pair intersect with each other.

In the control device of the present invention, preferably the first electrical field direction and the second electrical field direction intersect with each other orthogonally.

In the control device of the present invention, preferably at least a liquid or a gel containing at least an ion with an opposite charge to the charge of the substance is disposed on the flow path.

The control device of the present invention preferably further includes a detection means that separately detects a plurality of normal distributions of movement speed of the substance when a plurality of individuals of the substance are moved.

In order to address the above issues, a polynucleotide nucleotide sequence determination apparatus of the present invention includes the control device of the present invention.

Industrial Applicability

The present invention may be utilized in fields where it is necessary to control the movement speed of a substance with good precision. For example, the present invention may be employed in the next generation sequencers being pursued by the National Institutes for Health (NIH), and may be applied to next generation sequencers in which DNA amplification by PCR and chemical modification of DNA is not required. The present invention may also be applied to high sensitivity sensors for detecting a biomolecule such as an influenza virus or an allergen using one molecule thereof.

**Claims**

1.   A method of controlling movement speed of a substance, the method comprising:

   a movement process that moves a substance having a charge, along a first electrical field formed by a first electrode pair,
   wherein at least a portion of a movement path of the substance has a second electrical field formed by a second electrode pair in a direction intersecting with the first electrical field.

2.   The control method of claim 1, wherein a direction of the first electrical field and the direction of the second electrical field intersect with each other orthogonally.

3.   The control method of claim 1 or claim 2, wherein the substance is moved through at least one of a liquid or a gel containing at least an ion having an opposite charge to the charge of the substance.

4.   The control method of claim 3, further comprising a detection process that separately detects a plurality of normal distributions of movement speed of the substance when a plurality of separate units of the substance are moved.

5. The control method of any one of claim 1 to claim 4, wherein the substance is a nucleic acid, a protein, a pollen, a virus, a cell, an organic particle or an inorganic particle.

6. A device to control movement speed of a substance having a charge, the device comprising:

   a flow path provided between a first electrode pair; and
   a second electrode pair provided at at least a portion of the flow path,
   wherein a direction of a first electrical field formed by the first electrode pair and a direction of a second electrical field formed by the second electrode pair intersect with each other.

7. The control device of claim 6, wherein the first electrical field direction and the second electrical field direction intersect with each other orthogonally.

8. The control device of claim 6 or claim 7, wherein at least one of a liquid or a gel containing at least an ion having an opposite charge to the charge of the substance is disposed on the flow path.

9. The control device of claim 8, further comprising a detection means that separately detects a plurality of normal distributions of movement speed of the substance when a plurality of separate units of the substance are moved.

10. An apparatus to determine a nucleotide sequence of a polynucleotide, the apparatus comprising the control device of any one of claim 6 to claim 9.

# FIG.1

ELECTROOSMOTIC FLOW

SURFACE CHARGE

SiO2

GATE ELECTRODE

DEBYE LENGTH
(ELECTRICAL
DOUBLE LAYER)

# FIG.2

3-D FLOW PATH STRUCTURE
(SENSOR PORTION DEPTH
50nm, PILLAR ARRAY
REGION DEPTH 500nm)

ELECTRODE GAP/ FLOW
PATH WIDTH ABOUT 50nm

SEM IMAGE

1 $\mu$m

500nm

METAL-FREE SiO$_2$ PILLARS    5 $\mu$m

2 $\mu$m

FIG.3

L=200nm

H=50nm

w=60nm

FIG.4

FIG.5

# FIG.6

## (a)

$\lambda$-DNA, $V_{long}$ = 0.5 V, $V_{trans}$ = 0 V

## (b)

# FIG.7

## (a)

## (b)

FIG.8

# FIG.9

| | |
|---|---|
| **INTERNATIONAL SEARCH REPORT** | International application No. |
| | PCT/JP2013/051913 |

A.   CLASSIFICATION OF SUBJECT MATTER
*G01N27/447*(2006.01)i, *C12M1/00*(2006.01)i, *C12Q1/68*(2006.01)i

According to International Patent Classification (IPC) or to both national classification and IPC

B.   FIELDS SEARCHED

Minimum documentation searched (classification system followed by classification symbols)
G01N27/447, C12M1/00, C12Q1/68

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| X | JP 2005-501234 A  (Applera Corp.), 13 January 2005 (13.01.2005), paragraphs [0075] to [0079]; fig. 8b & US 2003/0075445 A1    & EP 1419112 A | 1–10 |

☐   Further documents are listed in the continuation of Box C.          ☐   See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 12 February, 2013 (12.02.13) | 26 February, 2013 (26.02.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011054631 W

**Non-patent literature cited in the description**

- **CLARKE, J. ; WU, H.-C. ; JAYASINGHE, L. ; PA-TEL, A ; REID, S. ; BAYLEY, H.** Continuous base identification for single-molecule nanopore DNA sequencing. *Nat. Nanotechnol.,* 2009, vol. 4, 265-270